# EUROPEAN PATENT SPECIFICATION

(11) **EP 2 908 237 B1**
(45) Date of publication and mention of the grant of the patent: **13.06.2018**
(21) Application number: 14155383.4
(22) Date of filing: 17.02.2014
(51) Int. Cl.: G06F 3/0488, G06F 19/00

(54) **Touch screen for an X-ray apparatus, according system and method for operating a touch screen**
Berührungsbildschirm für eine Röntgenvorrichtung, entsprechendes System und Verfahren zur Bedienung eines Berührungsbildschirms
Écran tactile pour un appareil à rayons X, conformément à un système et un procédé pour faire fonctionner un écran tactile

(43) Date of publication of application: 19.08.2015
(73) Proprietor: Agfa HealthCare N.V., 2640 Mortsel (BE)
(72) Inventor: Nebosis, Rainer, 81541 Munich (DE)

(56) References cited:
- WO-A1-00/02120
- US-A1- 2008 012 833

## Description

The present invention relates to a touch screen for an x-ray apparatus, a system comprising an x-ray apparatus and a touch screen as well as a method for operating a touch screen according to the independent claims.

Systems for medical x-ray examinations may comprise touch screens enabling a user to input data. In this way, when operating an x-ray apparatus, parameters and/or commands can be easily input by the user. Touch screens provided on conventional x-ray apparatuses, however, do not always meet requirements in terms of hygiene, safety and simplicity.

WO 00/02120 A1 relates to a method for cleaning a touch screen display in a touch screen device, wherein in a clean touch screen mode individual touches of the touch screen display are not registered, whereas the touch screen device exists the clean touch screen mode in response to input from the user, e.g., by depressing a key on a keyboard or simultaneously touching opposite corners of the touch screen display.

It is an object of the present invention to provide a touch screen for an x-ray apparatus, a system comprising an x-ray apparatus and a touch screen as well as a method for operating a touch screen allowing for a safe and simple operation of an x-ray apparatus, in particular in terms of hygiene requirements.

This object is achieved by a touch screen for an x-ray apparatus, a system comprising an x-ray apparatus and a touch screen as well as a method for operating a touch screen according to the independent claims.

The touch screen for an x-ray apparatus according to the invention comprises a controller designed to activate a first mode of the touch screen, wherein in the first mode the touch screen is sensitive to receive an input by means of touching the touch screen, and wherein the controller is further designed to activate a second mode of the touch screen, wherein in the second mode the touch screen is insensitive to any touching, or wherein in the second mode the touch screen is only sensitive to a predetermined termination input upon which the second mode is terminated.

The system according to the invention is defined in the appended claims.

In the method for operating a touch screen the touch screen is being switched between a first mode and a second mode, wherein in the first mode the touch screen is sensitive to receive an input by means of touching the touch screen, and wherein in the second mode the touch screen is insensitive to any touching or the touch screen is only sensitive to a predetermined termination input upon which the second mode is terminated.

The term "touchscreen" in the sense of the invention relates to an electronic visual display enabling a user to input data or commands by touching the screen with one or more fingers and/or a special stylus/pen. The input can occur through a simple touch, a multiple touch or a gesture. Accordingly, a touchscreen enables the user to interact directly with what is displayed on the screen.

The invention is based on the approach to enable the touchscreen to be operated in at least two different modes, namely a first mode, also regarded to be a "normal operation mode", in which the touchscreen is sensitive to touching such that the user can input data or commands by touching the screen, and a second mode, in which the touchscreen is insensitive to any touching. As a result, while being operated in the second mode, the touchscreen is insensitive to any input by means of touching, so that the touchscreen can be cleaned or disinfected by a user, e.g. by means of wetting and/or wiping, without the danger of an unintentional input of data or commands and without the need of shutting down the display for making it insensitive. Because in the second mode the touchscreen can advantageously be cleaned or disinfected, the second mode is also regarded to be a "cleaning mode".

Alternatively, in the second mode the touchscreen can be controlled such that it is sensitive and/or responsive only to a predetermined termination input of a user. Upon an input of a corresponding termination input by the user, the second mode is terminated. In other words, in the second mode the touchscreen is sensitive to touching so that a single touch, multiple touches and/or touching gestures of a user are detected. However, while being operated in the second mode, the touchscreen is not responsive with respect to any input of data or commands, except for an input which corresponds to a predetermined termination input, e.g. a predetermined gesture. As a result, while being operated in the second mode, the touchscreen is not responsive or not sensitive to touching, e.g. in form of a wiping, rubbing or scrubbing movement, applied during a usual cleaning and/or disinfecting procedure, so that the touchscreen can be conveniently cleaned or disinfected by a user, e.g. by means of wetting and/or wiping, rubbing or scrubbing, without the danger of an unintentional input of data or commands and without the need of shutting down the display. As soon as the cleaning or disinfecting procedure is terminated and/or the user wants to have the touchscreen back in the first mode, e.g. in order to be able to input data and/or commands relating to a further examination, the user can touch the touchscreen by a finger and/or stylus and execute one or more gestures corresponding to a predetermined termination input. Upon recognizing the user input to be a predetermined termination input, the second mode is terminated and the touch display is operated in the first mode.

All in all, a particular advantage of the touch screen according to the invention is that during activation of the second mode cleaning and disinfection procedures can be performed without risking unintentional input. Another advantage is that there is no need of shutting down the whole display and/or x-ray system for cleaning and disinfection procedures of the touch screen. These advantages become particularly important in the field of medical examinations, where hygiene rules have to be strictly observed, so that in the course of operating the x-ray system respective cleaning and disinfection procedures have to be performed rather frequently. For some cases even, after or before the examination of every patient appropriate cleaning and disinfection procedures have to be performed.

In summary, the invention allows for a safe and simple operation of an x-ray apparatus, in particular in terms of hygiene requirements.

According to a preferred embodiment, the controller is designed such that the second mode is activated for a predetermined time period, e.g. for 20, 40, 60, 90 or 120 seconds or even longer. Preferably, the controller is designed such that the operation mode of the touchscreen is automatically switched from the second mode to the first mode after the predetermined time period has lapsed. By this means, the touchscreen second mode is automatically terminated so that the user does not have to give an additional input for terminating the second mode and to switch back to the first mode which further simplifies operation.

According to a further preferred embodiment, the controller is designed such that during the activation of the second mode the controller effects the touch screen to display a mode indicator indicating that the touch screen is in the second mode. An advantage of this configuration is that the safety can be enhanced by enabling the operator to check easily whether the touch screen is in the first or second mode. By this means, the operator can be easily informed to refrain from continuing any cleaning and disinfection procedures in order to avoid unintentional input of data and/or commands in the first mode of the touchscreen, in particular after the predetermined time period for the activation of the second mode has lapsed.

The controller is designed such that during the activation of the second mode the controller calculates a remaining time for the second mode and the controller effects the touch screen to display a time indicator indicating the remaining time for the activation of the second mode. By this means, the operator is informed about the remaining time for possible cleaning and/or disinfecting activities so that he or she is able to stop wiping, rubbing or scrubbing gestures in due time in order to avoid unintentional input of data and/or commands.

Preferably, the time indicator comprises a number or a symbol, preferably a count-down token. For example, the displayed number of the time indicator corresponds to the duration of the remaining time. The displayed symbol or count-down token of the time indicator can be, e.g., in the form of an hour glass or a watch. In this way, the operator can easily and intuitively recognize the remaining time such that he or she is able to reliably stop the cleaning or disinfecting process in due time.

In a further preferred embodiment, the controller is designed such that during the activation of the first mode the controller effects the touch screen to display a time period selecting input field enabling a user or operator to input, in particular select, a time period, and upon a user input or user selection of the time period the controller is designed to use the inputted or selected time period as the predetermined time period during which the second mode of the touch screen is activated. An advantage of this configuration is that the time period of the activation of the second mode can be easily selected depending on the particular requirements of the cleaning and disinfection procedures to be performed. For example, the cleaning and disinfection procedures may be selected by the operator in dependence of the infectiousness of a patient examined.

According to another preferred embodiment, the controller is designed such that during the activation of the first mode the controller effects the touch screen to display an activation input field enabling a user to input an activation command and upon an input of an activation command, in particular upon touching the activation input field, the controller activates the second mode of the touch screen. By this means, the cleaning mode of the touch screen can be readily and reliably activated.

It is, moreover, preferred that the controller is designed such that during the activation of the second mode the controller effects the touch screen to display a keypad field enabling a user to input a sequence of characters, in particular numbers, the inputted sequence of characters is compared with a predetermined sequence of characters and, if the inputted sequence of characters corresponds to the predetermined sequence of characters, the second mode is terminated and/or the operation mode of the touchscreen is switched from the second mode back to the first mode. In this way, if the second mode has been selected erroneously, it can be safely terminated without the need of waiting for the end of the predetermined time period. Another advantage is that after completion of the cleaning and disinfection procedures the second mode can be terminated by the user without awaiting the end of the predetermined time period of the second mode. All in all, these embodiments further contribute to an enhanced safety and simplicity of operation of the x-ray apparatus.

According to another preferred embodiment, the controller is designed such that during the activation of the second mode the controller effects the touch screen to enable a user input in form of a gesture of the user, the input in form of a gesture of the user is compared with a predetermined gesture, for example an X- or Z-like wiping gesture, and, if the inputted gesture corresponds to the predetermined gesture, the second mode is terminated and/or the operation mode of the touchscreen is switched from the second mode back to the first mode. Preferably, the predetermined gesture corresponds to a gesture which is usually not used in cleaning procedures, for example a gesture having the form of "E", "G", "H", "X", "Y", "Z", "3", "4" or "5". Regarding this embodiment, the aforementioned elucidations regarding the advantages of a keypad apply accordingly. Therefore, by means of the present embodiment safety and simplicity of operation of the x-ray apparatus are further enhanced.

Preferably, the x-ray apparatus comprises an x-ray tube for generating x-rays, wherein the touch screen is provided on a casing of the x-ray tube. By this means, when positioning the x-ray tube before or after taking an x-ray of a patient the operator, who has to grasp the casing of the x-ray tube and/or a handle provided on the tube or the casing, is in proximity of the touchscreen and is therefore able to see information displayed on the touchscreen as well as to input data and/or commands.

Preferably, a position detector for detecting a position of the x-ray tube is provided, wherein the controller is designed to compare the detected position of the x-ray tube with a predetermined position of the x-ray tube and to activate the second mode in the case that the detected position of the x-ray tube corresponds to the predetermined position of the x-ray tube. For example, the predetermined position of the x-ray tube can be an idle and/or a waiting and/or a maintenance position in which the x-ray tube is not ready for taking x-rays. By means of the position detector such a predetermined position is detected and the second mode is activated automatically, so that the touchscreen provided on the casing of the x-ray tube is prepared for being cleaned and/or disinfected. Also this embodiment further contributes to an enhanced safety and simplicity of operation of the x-ray apparatus, in particular in view of hygiene requirements.

Preferably, an additional input unit is arranged separately from the touch screen, wherein the input unit comprises at least one mode changing switch selected from a group comprising: a switch for switching between the first and the second mode, a switch for starting the second mode, a switch for starting the first mode, a switch for ending or shortening the second mode, a switch for ending the first mode. By this means, the overall operation of the x-ray apparatus can be accelerated in a simple and reliable way by terminating the second mode whenever this is desired, e.g. when the second mode has been activated erroneously or when it is desired to shorten or terminate the second mode after the cleaning has been completed.

Further advantages, aspects and examples of the present invention will be apparent from the description of the following figures:
- Fig. 1: shows an example of a system comprising an x-ray apparatus and a touchscreen in a front view (left) and a side view (right);
- Fig. 2: shows a first example of a time sequence of screen content of a touchscreen being operated in a first mode, in a second mode and, again, in the first mode;
- Fig. 3: shows a second example of a time sequence of screen content of a touchscreen being operated in a first mode, in a second mode and, again, in the first mode; and
- Fig. 4: shows a preferred embodiment of the touch screen.

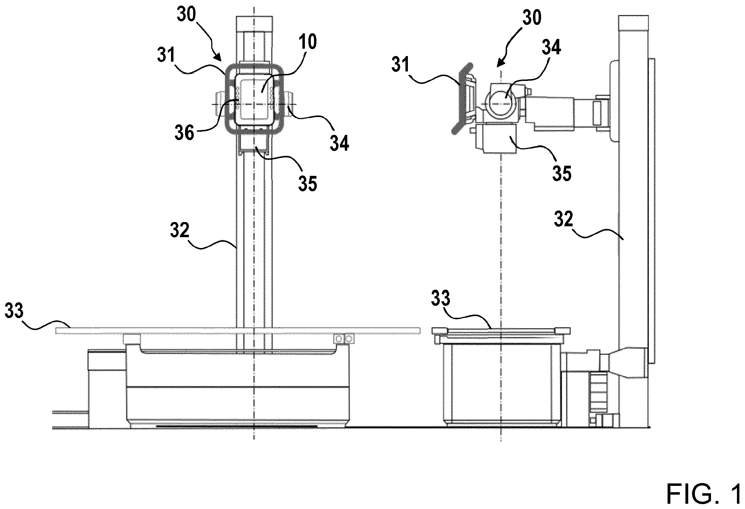

Figure 1 shows an example of an x-ray system with an x-ray apparatus 30 comprising a casing 34, in which an x-ray tube (not shown) is provided, and a collimator 35 for collimating x-ray radiation generated by the x-ray tube.

A touchscreen 10 is provided on or near to the casing 34 for displaying information and/or enabling a user or operator to input data and/or commands by touching the touchscreen 10, e.g. by means of a single touch, multiple touches or gestures. Preferably, input of data and/or commands is possible via touching the touchscreen with a finger, a hand and/or a stylus or pen.

One or more switches 36 are provided separately from the touchscreen 10. In the example shown, the switches 36 are arranged on a frame surrounding the touchscreen 10. Preferably, the switches 36 comprise one or more mechanical switches, e.g. pushbuttons. Alternatively or additionally, the switches 36 can comprise one or more touch sensitive switches.

Preferably, the casing 34 is provided with a handle 31 by means of which an operator can easily move the x-ray apparatus 30 to a desired position. To this purpose, the x-ray apparatus 30 is moveably mounted on a vertical shaft 32 such that the x-ray apparatus 30 can be shifted in a vertical direction, i.e. up and down, and/or tilted around at least one horizontal axis. Moreover, it is preferred that the vertical shaft 32 can be shifted in horizontal direction, i.e. left and right, such that the x-ray apparatus 30 can be positioned at different positions along x-ray table 33.

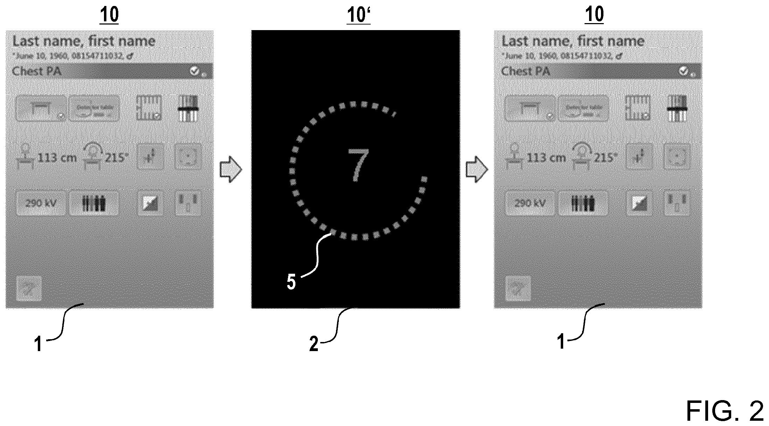

Figure 2 shows a first example of a time sequence of screen content of a touchscreen 10, 10' being operated in a first mode 1, in a second mode 2 and, again, in the first mode 1. In the first mode 1, the touch screen 10 is controlled such that by touching respective input fields on the touch screen 10 data and/or commands can be input or selected, respectively. In the second mode 2, the touch screen 10' is controlled such that either the touch screen 10' is insensitive to any touching or the touch screen 10' is only sensitive to a predetermined termination input. The touchscreen 10, 10' is controlled such that the second mode is activated only temporarily.

While being sensitive to data and/or command input in the first mode 1, data and/or command input is impossible during the second mode 2 of the touchscreen 10', except for a predetermined termination input.

While being operated in the second mode 2, the touchscreen 10' can be cleaned and/or disinfected by the operator, by means of usual wiping, rubbing or scrubbing movements or gestures, without the danger of unintentional data and/or command input. This is why the second mode 2 is also referred to as cleaning mode.

As shown in the present example, the touch screen 10' in the second mode 2 can preferably display a black background as a mode indicator indicating that the touch screen 10' is in the second mode 2. By means of displaying a black or a dark background, dirt, fingermarks and other contaminations can be easily recognized and reliably washed off by the operator.

The touch screen 10' in the second mode 2 displays a time indicator 5 indicating a remaining time for the activation of the second mode 2 by means of symbols and/or numbers. In the example shown in this figure, seven seconds are left for the cleaning and/or disinfecting procedure until the second mode 2 will be automatically terminated and the first mode 1, i.e. the normal operation mode, is activated again.

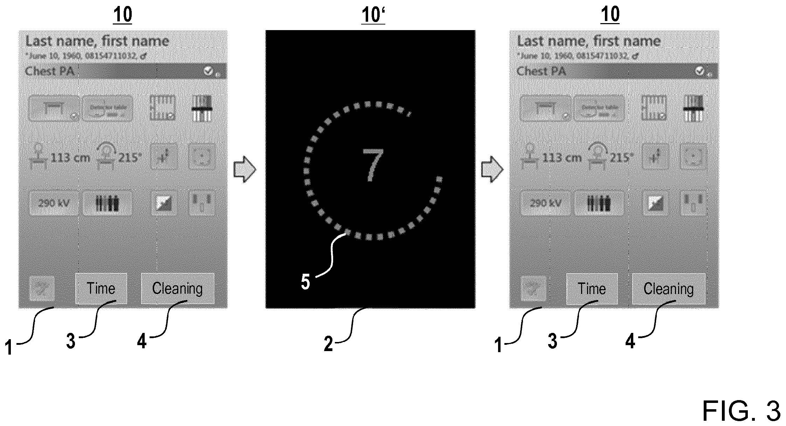

Figure 3 shows a second example of a time sequence of screen content of a touchscreen 10, 10' being operated in a first mode 1, in a second mode 2 and, again, in the first mode 1.

In the embodiment shown in this figure, the touch screen 10 in the first mode 1 preferably displays additional input or selection fields 3 and 4 for inputting or selecting parameters relating to the second mode 2.

For example, in the first mode 1 of the touchscreen 10 a time period selecting input field 3 is displayed for inputting or selecting a predetermined time period for the activation of the second mode 2. For example, upon touching the displayed field "Time" a drop-down list appears showing a number of predetermined time periods from which one time period can be selected by the user. Alternatively, an input field opens in which a value for the desired time period can be inputted. All in all, by this means, the time period of the activation of the second mode 2 can be easily adapted to different requirements in accordance with different medical examinations or patients.

Alternatively or additionally, the touch screen 10 in the first mode 1 can also display an activation input field 4, see displayed field "Cleaning", for starting the activation of the second mode 2. By this means, the operator can directly start the cleaning mode in a simple manner.

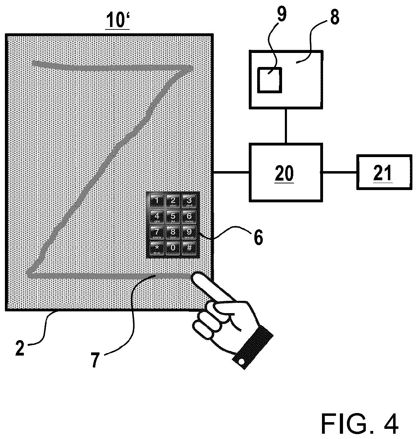

Figure 4 shows a preferred embodiment of the touch screen 10' comprising a controller 20, which is connected to the touch screen 10', for controlling the touchscreen 10' in the way outlined above.

According to a preferred embodiment, the second mode 2 of the touch screen 10' can be terminated by touching the touch screen 10' with a predetermined gesture. In the example given in figure 4, the predetermined gesture corresponds to a Z-like gesture executed by a finger of an operator.

In this embodiment, the touchscreen 10' is sensitive to touching but it is only responsive to a predetermined termination input in form of a predetermined gesture. That is, only in the case that the operator touches the touchscreen 10' being operated in the second mode 2 in a predetermined way, the controller 20 controls the touchscreen 10' such that the second mode 2 is terminated and/or the operation mode of the touchscreen 10' is switched from the second mode 2 to the first mode 1 (see fig. 2 and 3).

Preferably, in order to avoid unintentional termination of the second mode 2 during the cleaning or disinfecting activities, particular termination gestures of different complexity can be predetermined, for example an X- or Y-shaped gesture, such that it is rather unlikely that an operator executes such gesture while cleaning the touchscreen 10' by common wiping, rubbing or scrubbing movements.

Alternatively or additionally, the touch screen 10' in the second mode 2 displays, for example, a keypad field 6, wherein upon inputting a predetermined sequence of numbers and/or characters and/or a password into the displayed keypad field 6 on the touch screen 10' the second mode 2 can be terminated. By means of this, the safety can be further improved as only an operator knowing the predetermined sequence of numbers or the password can deliberately change the mode from the second mode 2 to the first mode 1 which is used for the normal operation of the x-ray apparatus 30 for medical examinations.

According to another preferred embodiment, an additional input unit 8 is provided which is connected with the controller 20 and which is preferably arranged separately from the touch screen 10'. The additional input unit 8 comprises at least one mode changing switch 9. By actuating the mode changing switch 9 a change of the operation mode of the touch screen 10' is effected via controller 20. Preferably, the at least one mode changing switch 9, which in particular can be a pushbutton, corresponds to at least one of the switches 36 provided on the frame around the touchscreen 10 as shown in figure 1. By separating the activation of the mode change from the touch screen 10', the risk of an unintentional mode change can be reliably and easily reduced.

Alternatively or additionally, the additional input unit 8 can comprise at least one of: a mode changing switch, like a switch for switching between the first mode 1 and the second mode 2; a switch for starting the second mode 2; a switch for starting the first mode 1; a switch for ending or shortening the second mode 2; a switch for ending the first mode 1. Preferably, regarding the aforementioned switches the elucidations as to the switches 36 shown in figure 1 apply accordingly.

Additionally or alternatively, a position detector 21 for detecting a position of the x-ray apparatus 30 and/or the x-ray tube accommodated in casing 34 (see figure 1) can be provided. For example, by means of the position detector 21 absolute coordinates of the x-ray apparatus 31 and/or an information regarding a relative position, e.g. a distance, between x-ray apparatus 31 and x-ray table 33 can be determined.

Preferably, the controller 20 is designed to compare the detected position of the x-ray tube or x-ray apparatus 30 with a predetermined position and to activate the second mode 2 in the case that the detected position corresponds to the predetermined position. For example, the predetermined position can be an idle and/or a waiting and/or a maintenance position in which the x-ray apparatus 30 is not ready for taking x-rays. By means of the position detector 21 such a predetermined position is detected and the second mode 2 is activated automatically.

Preferably, the predetermined position of the x-ray apparatus 30 corresponds to an ergonomically convenient position in which the touchscreen 10 can be easily accessed by the operator so that the touchscreen 10' can be cleaned and/or disinfected thoroughly and comfortably.

### Reference Signs

- 1: first mode of the touch screen
- 2: second mode of the touch screen
- 3: time period selecting input field
- 4: activation input field
- 5: time indicator
- 6: keypad field
- 7: Z-like wiping gesture
- 8: additional input unit
- 9: mode changing switch

- 10: touch screen in the first mode
- 10': touch screen in the second mode

- 20: controller
- 21: position detector

- 30: x-ray apparatus
- 31: handle
- 32: vertical shaft
- 33: x-ray table
- 34: casing of x-ray tube
- 35: collimator
- 36: separate switches

## Claims

1. A system comprising an x-ray apparatus (30) and a touch screen (10), wherein said touch screen (10) comprises a controller (20) designed to:
- activate a first mode (1) of the touch screen (10), wherein in the first mode (1) the touch screen (10) is sensitive to receive an input by means of touching the touch screen (10),
- activate a second mode (2) of the touch screen (10'), wherein in the second mode (2)
- the touch screen (10') is insensitive to any touching or
- the touch screen (10') is only sensitive to a predetermined termination input (7) upon which the second mode (2) is terminated, wherein said x-ray apparatus (30) comprises an x-ray tube for generating x-rays and a position detector for detecting a position of the x-ray tube, wherein the touch screen (10) is provided on or near to a casing (34) of the x-ray tube, and wherein the controller (20) is designed to:
- compare the detected position of the x-ray tube with a predetermined position of the x-ray tube, and to
- activate the second mode (2) in the case that the detected position of the x-ray tube corresponds to the predetermined position of the x-ray tube.

2. The system according to claim 1, wherein the controller (20) is designed such that the second mode (2) is activated for a predetermined time period.

3. The system according to claim 1 or 2, wherein the controller (20) is designed such that during the activation of the second mode (2) the controller (20) effects the touch screen (10') to display a mode indicator (5) indicating that the touch screen (10') is in the second mode (2).

4. The system according to any one of the claims 1 to 3, wherein the controller (20) is designed such that during the activation of the second mode (2) the controller (20) calculates a remaining time for the second mode (2) and the controller (20) effects the touch screen (10') to display a time indicator (5) indicating the remaining time of the activation of the second mode (2).

5. The system according to claim 4, wherein the time indicator (5) comprises a number or a symbol, preferably a count-down token.

6. The system according to any one of the claims 1 to 5, wherein the controller (20) is designed such that
- during the activation of the first mode (1) the controller (20) effects the touch screen (10) to display a time period selecting input field (3) enabling a user to input, in particular to select, a time period and
- upon a user input of a time period the controller (20) is designed to use the inputted, in particular selected, time period as the predetermined time period during which the second mode (2) of the touch screen (10') is activated.

7. The system according to any one of the claims 1 to 6, wherein the controller (20) is designed such that
- during the activation of the first mode (1) the controller (20) effects the touch screen (10) to display an activation input field (4) enabling a user to input an activation command and
- upon a user input of an activation command, in particular upon touching the activation input field (4), the controller (20) activates the second mode (2) of the touch screen (10').

8. The system according to any one of the claims 1 to 7, wherein the controller (20) is designed such that
- during the activation of the second mode (2) the controller (20) effects the touch screen (10') to display a keypad field (6) enabling a user to input a sequence of characters, in particular numbers,
- the inputted sequence of characters is compared with a predetermined sequence of characters and,
- if the inputted sequence of characters corresponds to the predetermined sequence of characters, the second mode (2) is terminated.

9. The system according to any one of the claims 1 to 8, wherein the controller (20) is designed such that
- during the activation of the second mode (2) the controller (20) effects the touch screen (10') to enable a user input in form of a gesture of the user and
- the input in form of a gesture of the user is compared with a predetermined gesture, in particular a Z-like wiping gesture (7), and
- if the inputted gesture corresponds to the predetermined gesture, the second mode (2) is terminated.

10. The system according to any of the claims 1 to 5 comprising an additional input unit (8) being arranged separately from the touch screen (10) and comprising at least one mode changing switch (9) selected from a group comprising:
- a switch for switching between the first mode (1) and the second mode (2),
- a switch for starting the second mode (2),
- a switch for starting the first mode (1),
- a switch for terminating or shortening the second mode (2),
- a switch for terminating the first mode (1).

## Patentansprüche

1. Ein System, umfassend einen Röntgenapparat (30) und einen Berührungsbildschirm (10), wobei der Berührungsbildschirm (10) eine Steuereinheit (20) umfasst, die so ausgelegt ist, dass
- sie einen ersten Modus (1) des Berührungsbildschirms (10) aktiviert, wobei im ersten Modus (1) der Berührungsbildschirm (10) zum Empfang einer Eingabe durch Berühren des Berührungsbildschirms (10) empfindlich ist,
- sie einen zweiten Modus (2) des Berührungsbildschirms (10') aktiviert, wobei im zweiten Modus (2)
- der Berührungsbildschirm (10') unempfindlich für irgendwelches Berühren ist oder
- der Berührungsbildschirm (10') nur für eine vorgegebene Abbrucheingabe (7), wobei der zweite Modus (2) beendet wird, empfindlich ist,
wobei der Röntgenapparat (30) eine Röntgenröhre zur Erzeugung von Röntgenstrahlen und einen Positionsdetektor zum Detektieren einer Position der Röntgenröhre umfasst,
wobei der Berührungsbildschirm (10) auf oder nahe an einem Gehäuse (34) der Röntgenröhre angeordnet ist, und
wobei die Steuereinheit (20) so ausgelegt ist, dass
- sie die detektierte Position der Röntgenröhre mit einer vorgegebenen Position der Röntgenröhre vergleicht, und
- den zweiten Modus (2) dann aktiviert, wenn die detektierte Position der Röntgenröhre der vorgegebenen Position der Röntgenröhre entspricht.

2. System nach Anspruch 1, wobei die Steuereinheit (20) so ausgelegt ist, dass der zweite Modus (2) für eine vorgegebene Zeitdauer aktiviert wird.

3. System nach Anspruch 1 oder 2, wobei die Steuereinheit (20) so ausgelegt ist, dass während der Aktivierung des zweiten Modus (2) die Steuereinheit (20) den Berührungsbildschirm (10') dazu veranlasst, eine Modusanzeige (5), die anzeigt, dass sich der Berührungsbildschirm (10') im zweiten Modus (2) befindet, anzuzeigen.

4. System nach einem der Ansprüche 1 bis 3, wobei die Steuereinheit (20) so ausgelegt ist, dass während der Aktivierung des zweiten Modus (2) die Steuereinheit (20) eine Restzeit für den zweiten Modus (2) berechnet und die Steuereinheit (20) den Berührungsbildschirm (10') dazu veranlasst, eine Zeitanzeige (5), die die Restzeit der Aktivierung des zweiten Modus (2) zeigt, anzuzeigen.

5. System nach Anspruch 4, wobei die Zeitanzeige (5) eine Ziffer oder ein Symbol, vorzugsweise ein Countdown-Token, umfasst.

6. System nach einem der Ansprüche 1 bis 5, wobei die Steuereinheit (20) so ausgelegt ist, dass
- während der Aktivierung des ersten Modus (1) die Steuereinheit (20) den Berührungsbildschirm (10) dazu veranlasst, ein Zeitdauerauswahleingabefeld (3) anzuzeigen, mit dem ein Benutzer eine Zeitdauer eingeben, insbesondere auswählen, kann, und
- nach Benutzereingabe einer Zeitdauer die Steuereinheit (20) so ausgelegt ist, dass sie dann die eingegebene, insbesondere ausgewählte, Zeitdauer als die vorgegebene Zeitdauer, während der der zweite Modus (2) des Berührungsbildschirms (10') aktiviert wird, benutzt.

7. System nach einem der Ansprüche 1 bis 6, wobei die Steuereinheit (20) so ausgelegt ist, dass
- während der Aktivierung des ersten Modus (1) die Steuereinheit (20) den Berührungsbildschirm (10) dazu veranlasst, ein Aktivierungseingabefeld (4), mit dem ein Benutzer einen Aktivierungsbefehl eingeben kann, anzuzeigen, und
- nach Benutzereingabe eines Aktivierungsbefehls, insbesondere nach Berühren des Aktivierungseingabefeldes (4), die Steuereinheit (20) den zweiten Modus (2) des Berührungsbildschirms (10') aktiviert.

8. System nach einem der Ansprüche 1 bis 7, wobei die Steuereinheit (20) so ausgelegt ist, dass
- während der Aktivierung des zweiten Modus (2) die Steuereinheit (20) den Berührungsbildschirm (10') dazu veranlasst, einen Ziffernblock (6), mit dem ein Benutzer eine Sequenz von Zeichen, insbesondere Ziffern, eingeben kann, anzuzeigen,
- die eingegebene Sequenz von Zeichen mit einer vorgegebenen Sequenz von Zeichen verglichen wird, und
- falls die eingegebene Sequenz von Zeichen der vorgegebenen Sequenz von Zeichen entspricht, der zweite Modus (2) beendet wird.

9. System nach einem der Ansprüche 1 bis 8, wobei die Steuereinheit (20) so ausgelegt ist, dass
- während der Aktivierung des zweiten Modus (2) die Steuereinheit (20) den Berührungsbildschirm (10') dazu veranlasst, eine Benutzereingabe in Form einer Bewegung des Benutzers zu ermöglichen, und
- die Eingabe in Form einer Bewegung des Benutzers mit einer vorgegebenen Bewegung, insbesondere einer Z-artigen Streichbewegung (7), verglichen wird, und
- falls die eingegebene Bewegung der vorgegebenen Bewegung entspricht, der zweite Modus (2) beendet wird.

10. System nach einem der Ansprüche 1 bis 5, umfassend eine zusätzliche Eingabeeinheit (8), die getrennt vom Berührungsbildschirm (10) angeordnet ist und mindestens einen Moduswechselschalter (9) umfasst, ausgewählt aus einer Gruppe bestehend aus:
- einem Schalter, mit dem zwischen dem ersten Modus (1) und dem zweiten Modus (2) geschaltet wird,
- einem Schalter zum Starten des zweiten Modus (2),
- einem Schalter zum Starten des ersten Modus (1),
- einem Schalter zum Abbrechen oder Verkürzen des zweiten Modus (2),
- einem Schalter zum Abbrechen des ersten Modus (1).

## Revendications

1. Système comprenant un appareil à rayons X (30) et un écran tactile (10), ledit écran tactile (10) comprenant une unité de contrôle (20) conçue de façon à
- activer un premier mode (1) de l'écran tactile (10), où, dans ledit premier mode (1), l'écran tactile (10) est sensible à recevoir une entrée en touchant l'écran tactile (10),
- activer un deuxième mode (2) de l'écran tactile (10'), où, dans ce deuxième mode (2)
- l'écran tactile (10') est insensible à tout toucher ou
- l'écran tactile (10') n'est sensible qu'à une entrée d'arrêt prédéfinie (7) permettant de terminer le deuxième mode (2), ledit appareil à rayons X (30) comprenant un tube à rayons X servant à générer des rayons X et un détecteur de position servant à détecter une position du tube à rayons X,
ledit écran tactile (10) étant disposé sur ou à proximité d'un boîtier (34) du tube à rayons X, et
ladite unité de contrôle (20) étant conçue de façon à
- comparer la position détectée du tube à rayons X avec une position prédéfinie du tube à rayons X, et
- activer le deuxième mode (2) dans le cas où la position détectée du tube à rayons X correspond à la position prédéfinie du tube à rayons X.

2. Système selon la revendication 1, **caractérisé en ce que** l'unité de contrôle (20) est conçue de façon à activer le deuxième mode (2) pour une durée de temps prédéfinie.

3. Système selon la revendication 1 ou 2, **caractérisé en ce que** l'unité de contrôle (20) est conçue de façon à ce que, lors de l'activation du deuxième mode (2), l'unité de contrôle (20) amène l'écran tactile (10') à afficher un indicateur de mode (5) affichant que l'écran tactile (10') se trouve dans le deuxième mode (2).

4. Système selon l'une quelconque des revendications 1 à 3, **caractérisé en ce que** l'unité de contrôle (20) est conçue de façon à ce que, lors de l'activation du deuxième mode (2), l'unité de contrôle (20) calcule un temps restant pour le deuxième mode (2) et l'unité de contrôle (20) amène l'écran tactile (10') à afficher un indicateur de temps (5) affichant le temps restant de l'activation du deuxième mode (2).

5. Système selon la revendication 4, **caractérisé en ce que** l'indicateur de temps (5) comprend un chiffre ou un symbole, de préférence une zone de décompte.

6. Système selon l'une quelconque des revendications 1 à 5, **caractérisé en ce que** l'unité de contrôle (20) est conçue de façon à ce que
- lors de l'activation du premier mode (1), l'unité de contrôle (20) amène l'écran tactile (10) à afficher un champ d'entrée de sélection de durée de temps (3) permettant à un utilisateur d'entrer, en particulier de sélectionner, une durée de temps, et
- suite à une entrée d'une durée de temps effectuée par l'utilisateur, l'unité de contrôle (20) est conçue de façon à utiliser ensuite la durée de temps entrée, en particulier sélectionnée, comme la durée de temps prédéfinie lors de laquelle le deuxième mode (2) de l'écran tactile (10') est activé.

7. Système selon l'une quelconque des revendications 1 à 6, **caractérisé en ce que** l'unité de contrôle (20) est conçue de façon à ce que
- lors de l'activation du premier mode (1), l'unité de contrôle (20) amène l'écran tactile (10) à afficher un champ d'entrée d'activation (4) permettant à un utilisateur d'entrer une commande d'activation, et
- suite à une entrée d'une commande d'activation effectuée par l'utilisateur, en particulier après que le champ d'entrée d'activation (4) a été touché, l'unité de contrôle (20) active le deuxième mode (2) de l'écran tactile (10').

8. Système selon l'une quelconque des revendications 1 à 7, **caractérisé en ce que** l'unité de contrôle (20) est conçue de façon à ce que
- lors de l'activation du deuxième mode (2), l'unité de contrôle (20) amène l'écran tactile (10') à afficher un pavé numérique (6) permettant à un utilisateur d'entrer une séquence de caractères, en particulier des chiffres,
- la séquence de caractères entrée soit comparée avec une séquence de caractères prédéfinie, et
- dans le cas où la séquence de caractères entrée correspond à la séquence de caractères prédéfinie, le deuxième mode (2) soit terminé.

9. Système selon l'une quelconque des revendications 1 à 8, **caractérisé en ce que** l'unité de contrôle (20) est conçue de façon à ce que
- lors de l'activation du deuxième mode (2), l'unité de contrôle (20) amène l'écran tactile (10') à permettre une entrée effectuée par un utilisateur sous forme d'un geste de l'utilisateur, et
- l'entrée sous forme d'un geste de l'utilisateur soit comparée avec un geste prédéfini, en particulier un geste de marquage en forme de Z (7), et
- dans le cas où le geste entré correspond au geste prédéfini, le deuxième mode (2) soit terminé.

10. Système selon l'une quelconque des revendications 1 à 5, comprenant une unité d'entrée supplémentaire (8) disposée séparément de l'écran tactile (10) et comprenant au moins un commutateur de changement de mode (9) choisi parmi un groupe composé:
- d'un commutateur permettant de commuter entre le premier mode (1) et le deuxième mode (2),
- d'un commutateur permettant de démarrer le deuxième mode (2),
- d'un commutateur permettant de démarrer le premier mode (1),
- d'un commutateur permettant de terminer ou de raccourcir le deuxième mode (2),
- d'un commutateur permettant de terminer le premier mode (1).
